# EUROPEAN PATENT SPECIFICATION

(11) **EP 2 692 864 B1**
(45) Date of publication and mention of the grant of the patent: **12.09.2018**
(21) Application number: 12765903.5
(22) Date of filing: 27.03.2012
(51) Int. Cl.: C12N 15/63, C12N 15/64, C12N 15/867, C12N 15/86

(54) **VECTOR FOR FOREIGN GENE INTRODUCTION AND METHOD FOR PRODUCING VECTOR TO WHICH FOREIGN GENE HAS BEEN INTRODUCED**
VEKTOR ZUR EINFÜHRUNG FREMDER GENE UND VERFAHREN ZUR HERSTELLUNG VON VEKTOREN MIT DARIN EINGEFÜHRTEN FREMDEN GENEN
VECTEUR POUR L'INTRODUCTION D'UN GÈNE ÉTRANGER ET PROCÉDÉ DE PRODUCTION DE VECTEUR DANS LEQUEL UN GÈNE ÉTRANGER A ÉTÉ INTRODUIT

(30) Priority: 30.03.2011 JP 2011075273
(43) Date of publication of application: 05.02.2014
(73) Proprietor: National University Corporation University Of Toyama, Toyama-shi, Toyama 930-8555 (JP)
(72) Inventor: HORII, Masae, Toyama-shi Toyama 930-0194 (JP); KISHI, Hiroyuki, Toyama-shi Toyama 930-0194 (JP); KOBAYASHI, Eiji, Toyama-shi Toyama 930-0194 (JP); OZAWA, Tatsuhiko, Toyama-shi Toyama 930-0194 (JP); MURAGUCHI, Atsushi, Toyama-shi Toyama 930-0194 (JP)
(74) Representative: dompatent von Kreisler Selting Werner - Partnerschaft von Patent- und Rechtsanwälten mbB
(86) International application number: PCT/JP2012/057875
(87) International publication number: WO 2012/133376

(56) References cited:
- JP-A- 2002 526 083
- Biosettia Inc.: "Lentiviral miRNA Expression Lentiviral Transduction for Specific miRNA Expression", , 1 April 2010 (2010-04-01), pages 1-10, XP055157635, Retrieved from the Internet: URL:http://biosettia.com/download/protocol s/biosettia-mirna-lentivirus-manual.pdf [retrieved on 2014-12-09]
- LU ET AL: "Seamless cloning and gene fusion", TRENDS IN BIOTECHNOLOGY, ELSEVIER PUBLICATIONS, CAMBRIDGE, GB, vol. 23, no. 4, 1 April 2005 (2005-04-01), pages 199-207, XP027778078, ISSN: 0167-7799 [retrieved on 2005-04-01]
- MATRAI JANKA ET AL: "Recent Advances in Lentiviral Vector Development and Applications", MOLECULAR THERAPY, NATURE PUBLISHING GROUP, GB, vol. 18, no. 3, 19 January 2010 (2010-01-19), pages 477-490, XP009143103, ISSN: 1525-0016, DOI: 10.1038/MT.2009.319 [retrieved on 2010-01-19]
- 'Lentiviral Vectors for Gene Expression, Catalog number: cDNA-pLV##' BIOSETTIA INC. February 2010, pages 1 - 11, XP055124322
- DEROOSE, CM. ET AL.: 'Noninvasive monitoring of long-term lentiviral vector-mediated gene expression in rodent brain with bioluminescence imaging.' MOL. THER. vol. 14, no. 3, September 2006, pages 423 - 431, XP005597057
- KOSAKA, Y. ET AL.: 'Lentivirus-based gene delivery in mouse embryonic stem cells.' ARTIF. ORGANS vol. 28, no. 3, March 2004, pages 271 - 277, XP055124324
- THOMASON, L. ET AL.: 'Recombineering: genetic engineering in bacteria using homologous recombination.' CURR. PROTOC. MOL. BIOL. April 2007, pages 1 - 24, XP002531244
- MA, H. ET AL.: 'Plasmid construction by homologous recombination in yeast.' GENE vol. 58, no. 2-3, 1987, pages 201 - 216, XP025705444
- CHINO, A. ET AL.: 'Plasmid construction using recombination activity in the fission yeast Schizosaccharomyces pombe.' PLOS ONE vol. 5, no. 3, 2010, page E9652, XP055095577

## Description

### Technical Field

This invention relates to a preparation method of a vector in which a foreign gene has been introduced and its use.

### Background Art

Generally, restriction enzyme is usually used for introduction of desired gene into vector. Specifically, a vector is cleaved with a specific restriction enzyme at multicloning site and a nucleic acid fragment of desired gene having the same restriction enzyme site with such specific restriction enzyme is introduced to such cleaved site. The following method for introduction of a desired gene into vector has been recently developed and is commonly used; preparing a nucleic acid fragment which contains a desired gene having two different sequences at either end of the fragment, each of which is homologous with corresponding sequence existing in vector; and causing homologous recombination between the above two sequences in vector and in nucleic acid fragment. (Zhang Y, Muyrers J.P.P., Testa G and Stewart A.F. DNA cloning by homologous recombination in E. Coli. Nature Biotechnology 18: 1314-1317, 2000, and Quinn Lu, Seamless cloning and gene fusion. TRENDS in Biotechnology 23: 199-207, 2005)

Thomason, L. et al.: "Recombineering: genetic engineering in bacteria using homologous recombination", Current Protocols in Molecular Biology, April 2007, pages 1 - 24 discloses strategies and protocols with respect to homologous recombination in bacteria.

Chino, A. et al.: "Plasmid construction using recombination activity in the fission yeast Schizosaccharomyces pombe, PLoS ONE, Vol. 5, No. 3, 2010, page E9652 discloses transfer of the gap-repair cloning (GRC) technique from *Saccharomyces Cerevisiae* to *Schizosaccharomyces pombe.*

Ma, H. et al.: "Plasmid construction by homologous recombination in yeast", Gene, Vol. 58, No. 2 - 3, 1987, pages 201 - 216 discloses a convenient method for the construction of new plasmids relying on interchanging parts of plasmids by homologues Recombination in *Saccharomyces cerevisiae.*

Biosettia Inc. provides lentiviral vectors for gene expression (cf. catalogue number cDNA-pLV##, February 2010, disclosing vectors as well as respective protocols) and a collection of human lenti-miRNA (cf. catalogue number: mir-LV### of April 2010, disclosing an expression system and respective protocols).

Kosaha, Y. et al.: "Lentivirus-based gene delivery in mouse embryonic stem cells", Artificial Organs, Vol. 28, No. 3, 2004, pages 271 - 277 discloses a study of the efficacy of lentivirals-based gene delivery into mouse embryonic stem cells by GFP expression assay or X-gal staining.

Matrai, J. et al.: "Recent advances in lentiviral vector development and applications", Molecular Therapy, Vol. 18, No. 3, pages 477 - 490 reviews the role of lentiviral vectors in the development of fundamental biological research, functional genomics and gene therapy.

Droose, C. M. et al.: "Non-invasive monitoring of long-term lentiviral vector-mediated gene expression in rodent brain with bioluminescence imaging", Molecular Therapy, Vol. 14, No. 3, September 2006, pages 423 - 431 discloses construction of HIV type I lentiviral vectors and their bioluminescence imaging after gene transfer into the mouse brain.

### Summary of the Invention

### Problem to be solved by the Invention

The above method using homologous recombination is very simple, easy-to-use and convenient method in terms of introduced desired genes into a vector without depending on the restriction enzyme sites. However, unexpected homologous recombination is induced at high rates inside the vector other than a certain region, in case that multiple sequences homologous exist in the vector to be introduced foreign gene by homologous recombination. As the result, it is very difficult or almost impossible to obtain the vector into which desired gene is introduced, when introduction of desired gene to such vector is tried using homologous recombination method, because homologous recombination inside the vector frequently occurs. This is shown in a result of experiment mentioned in non-patent literature 1.

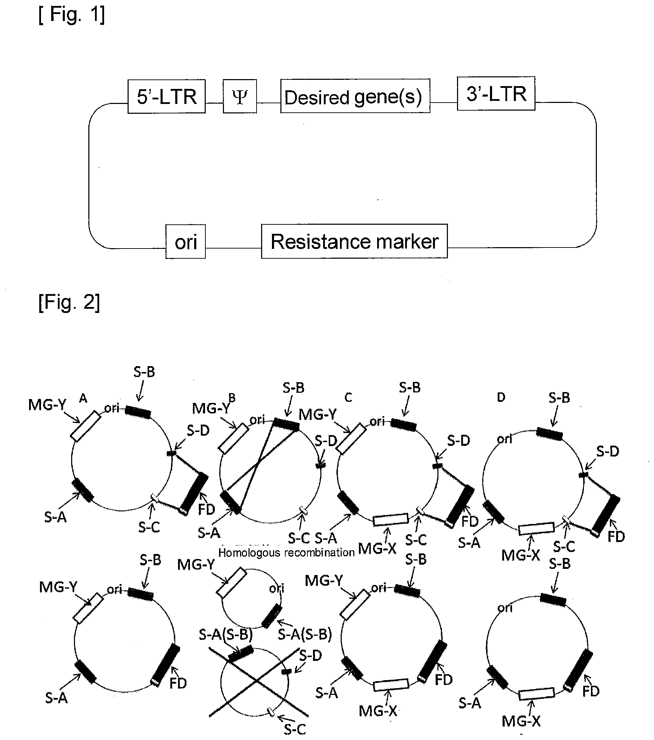

Retroviral vectors or lentiviral vector (a kind of retroviral vectors) is one of typical example that two sequences homologous with one another exist in a certain region in the vector other than the region to be intended to introduce foreign gene by homologous recombination. Retroviral vector and lentiviral vector are used for research and therapeutic purposes in various fields because these vectors are possible to introduce significantly a gene into animal cell having a characteristic of difficulty in gene introduction. Retroviral vector and lentiviral vector have long terminal repeat (LTR) being homologous sequence in two regions as shown in FIG. 1. Desired gene is usually introduced into the region shown in FIG. 1 using restriction enzyme site. Desired gene was almost impossible to be introduced into conventional retroviral vectors by homologous recombination as shown in FIG. 2A, because 5'-LTR and 3'-LTR of homologous sequence exist and homologous recombination is frequently caused between these sequences like FIG. 2B.

Purpose of this invention is to provide a new vector technology by which the problem of the above conventional technology is solved and foreign genes can be introduced by homologous recombination. In particular, this invention is to provide means that can effectively select a vector in which a foreign gene has been introduced in a method of introducing a foreign gene for a method of introducing a foreign gene into a vector containing multiple sequences homologous with one another at a certain region other than an intended region into which the foreign gene is introduced by homologous recombination.

### Means for Solving Problem

We, inventors examined in order to solve the above problem intensively. In the method of introducing a foreign gene by homologous recombination into the vector containing multiple sequences homologous with one another in a region where introduction of the foreign gene by homologous recombination is not expected, it was found that the use of a vector containing two sequences homologous with two sequences for introducing foreign genes by homologous recombination and a marker gene which is arranged in an adjacent region located between one side of sequence and another side of sequence for introducing a foreign gene was resulted in effective selection and accession of a vector in which a foreign gene has been introduced. This invention has been accomplished on the basis of the above findings.

That is to say, this disclosure relates to the followings;
[1] A vector containing a replication origin, sequence A, marker gene X, sequences C and D for introducing a foreign gene by homologous recombination, and sequence B that is homologous with said sequence A, in that order, wherein said sequences C and D are directly or indirectly adjacent, and for use in introducing the foreign gene between said adjacent sequences C and D.
[2] The vector according to [1], wherein the vector is a retroviral vector.
[3] The vector according to [1] or [2], wherein the vector is a lentiviral vector.
[4] The vector according to any one of [1] to [3], wherein said sequence A is 3 'LTR of a retroviral genome and said sequence B is 5' LTR of a retroviral genome.
[5] The vector according to any one of [1] to [3], wherein said sequence A is 5' LTR contained in a retroviral genome and said sequence B is 3' LTR contained in a retroviral genome.
[6] The vector according to any one of [1] to [5], wherein marker gene X is a fluorescence protein gene or a drug-resistant gene.
[7] The vector according to any one of [1] to [5], wherein marker gene X is a gene encoding luciferase or β-galactosidase.
[8] The vector according to any one of [1] to [7], wherein the replication origin and sequence A are indirectly adjacent, and marker gene Y different from marker gene X is contained between said adjacent replication origin and sequence A.
[9] The vector according to any one of [1] to [7], wherein the replication origin and sequence B are indirectly adjacent, and marker gene Y different from marker gene X is contained between the said adjacent replication origin and sequence B.
[10] The vector according to [8] or [9], wherein marker gene Y is a fluorescence protein gene or a drug-resistant gene.
[11] The vector according to [8] or [9], wherein marker gene Y is a gene encoding luciferase or β-galactosidase.
[12] The vector according to any one of [1] to [11], wherein the replication origin is a replication origin of an organism selected from Escherichia coli, bacteriophage, Saccharomyces cerevisiae and Schizosaccharomyces pombe.
   This invention relates to the following:
[13] A method of producing a recombinant retroviral vector in which a foreign gene has been introduced between said adjacent sequences C and D, comprising the steps of;
   (1) preparing a retroviral vector according to any one of [1] to[12];
   (2) preparing a nucleic acid fragment containing sequence C' that is homologous with said sequence C, a foreign gene, and sequence D' that is homologous with said sequence D, in that order;
   (3) by exposing said retroviral vector and said nucleic acid fragment to conditions to cause homologous recombination, generating a recombinant retroviral vector in which the foreign gene has been introduced between said adjacent sequences C and D, and forming a host that may contain the recombinant retroviral vector in which the foreign gene has been introduced between said adjacent sequences C and D;
   (4) culturing the host obtained in Step (3), and selecting from the culture a host containing the recombinant retroviral vector in which the foreign gene has been introduced between said adjacent sequences C and D by identifying the expression of marker gene X as an indicator; and
   (5) extracting form the host selected in Step (4) the recombinant retroviral vector in which the foreign gene has been introduced between said adjacent sequences C and D, wherein said sequence A is 3' LTR contained in a retroviral genome and said sequence B is 5' LTR contained in a retroviral genome, or said sequence A is 5' LTR contained in a retroviral genome and said sequence B is 3' LTR contained in a retroviral genome.
[14] The method according to [13], wherein said Step (3) is performed by mixing said retroviral vector and said nucleic acid fragment with an enzyme for homologous recombination to obtain a homologous recombination product, and transforming a host with the obtained product.
[15] The method according to [13], wherein said Step (3) is performed by obtaining a gene encoding an enzyme for homologous recombination and a host containing said nucleic acid fragment and said vector, and expressing the gene encoding an enzyme for homologous recombination in the obtained host.

### Effect of the Invention

According to this disclosure, in a method for introducing a foreign gene by homologous recombination to a vector having multiple sequences homologous with one another, a vector in which the foreign gene has been introduced is effectively selected and obtained. Especially, according to this invention, in a method for introducing a foreign gene by homologous recombination to a vector that is a retroviral vector or lentiviral vector, a vector in which the foreign gene has been introduced is effectively selected and obtained.

### Brief Description of Drawings

[FIG. 1] FIG. 1 shows the basic structure of retroviral vector and lentiviral vector. (LTR long terminal repeat): This is indispensable for expression and preparation of virus genome.
   5' -LTR and 3'- LTR have homologous sequences.
   ψ: Sequence necessary packaging of virus genome into virus particle.
   ori: Replication origin of DNA. This is indispensable for replication of DNA in Escherichia coli.
   A resistant marker: This is necessary for selective growth of Escherichia coli containing plasmid DNA.
[FIG. 2] FIG. 2 shows the method to insert desired DNA by homologous recombination into a vector having homologous sequence inside such vector. S-A and S-B show homologous region in the vector. MG-Y shows marker gene Y (selective marker Y). S-C and S-D show homologous recombination sequence for introducing a desired DNA (a foreign gene) into a vector. FD shows desired DNA (a foreign gene). MG-X shows marker gene X (selective marker X).
[FIG. 3] FIG. 3 shows location of marker gene (a selective marker) in the vector of this invention. Each code symbol is the same with FIG. 2.
[FIG. 4] FIG. 4 shows a result of comparative example and practical example.
[FIG. 5] FIG. 5 shows the structure of retroviral vector for conventional antibody gene expression.
[FIG. 6] FIG. 6 shows a preparation method of insert DNA which is possible to be inserted into a vector by homologous recombination in the comparative example.
[FIG. 7] FIG. 7 shows the structure of retroviral vector for TCR gene expression in this invention.
[FIG. 8] FIG. 8 shows a preparation method of insert DNA which can be inserted into a vector by homologous recombination in the practical example.
[FIG. 9] FIG. 9 shows other example of vector by this invention (a structure shown in FIG. 3B).
[FIG. 10] FIG. 10 shows a result of the practical example of insertion of human-derived desired gene by homologous recombination into a vector of this invention.
[FIG. 11] FIG. 11 shows gene sequence analyzed.
[FIG. 12] FIG. 12 shows an analysis result by flow cytometry.

### Mode for Carrying Out the Invention

### (The vector is not part of the invention but can be used according to the method of the invention as in claim 1)

This disclosure relates to a vector using for introducing a foreign gene between adjacent 2 sequences C and D below, wherein the vector contains replication origin, sequence A, marker gene X, 2 sequences C and D for introducing the foreign gene by homologous recombination and sequence B homologous with the above sequence A in that order, and the above sequence C and D are directly or indirectly adjacent.

In this invention, "being directly adjacent" means 2 sequences are directly linked each other and other sequence does not exist on the adjacent site of 2 sequences. On the other hand, "being indirectly adjacent" means 2 sequences are linked through any other sequence.

Characteristic of a vector in this disclosure is to contain replication origin, sequence A, marker gene X, 2 sequences C and D for introducing foreign gene by homologous recombination, in that order.

In this invention, replication origin means an initiation site of replication of nucleic acid which composes a vector in this invention. The replication origin includes that of a bacteriophage, a prokaryote (including bacteria) and eukaryotic organism. In this disclosure, a vector is preferable to do a replication origin of bacterium and is more preferable to include a replication origin of Escherichia coli. In this disclosure, a vector is possible to include a replication origin of bacteriophage or yeast (e.g., Saccharomyces cerevisiae, Schizosaccharomyces pombe).

As is described in the above, the vector of this disclosure has sequence A and sequence B, in its inside, which are homologous with each other. Sequence A and sequence B is a gene sequence existing without a region to be intended to introduce a foreign gene by homologous recombination. As an example of the vector containing such sequences A and B which are homologous with each other, a retroviral vector is included. To further specify, lentiviral vector is an example of retroviral vector.

However, it is not intended to limit these vectors as a vector having sequences A and B homologous with one another. Sequences A and B homologous with one another means sequences possible to cause recombination by homologous recombination described below. The number of base in sequences A and B is not specially restricted; for example, a sequence so as to cause homologous recombination between each other in at least a part of sequences A and B. The number of base necessary for causing homologous recombination varies according to tools (e.g., enzyme for recombination), for example; the number of base can be possible in a range of 2 to 600. Or, the above "homologous with one another" means that sequences A and B have base sequences similar to each other as much as homologous recombination can be caused. The homology of sequences A and B in a range possible to cause homologous recombination is, for example, 70% or more, 80% or more, 90% or more, 95% or more, 96% or more, 97% or more, 98% or more, 99% or more, 100%.

Two sequences C and D are sequences which are used for introducing a foreign gene by homologous recombination. Two sequences C and D are homologous with base sequences of nucleic acid fragment 5' end and 3' end containing a foreign gene which are material for introducing foreign gene into vector of this invention. The number of base of two sequences C and D varies according to tools (e.g., enzyme for recombination) possible to use for homologous recombination, for example; the number of base is in a range of 2 to 600. However, it is not intended to limit the number of base within this range. In a case that sequence C is homologous with base sequence of the above nucleic acid fragment 5' end, sequence D is homologous with base sequence of the above nucleic acid 3' end. On the other hand, in a case sequence C is homologous with sequence of the above nucleic acid fragment 3' end, sequence D is homologous with base sequence of the above nucleic acid 5' end. And "homologous" means that sequences C and D are similar to base sequence of the above nucleic acid fragment 5' end and 3' end as much as homologous recombination between sequences C and D in this invention and the above nucleic acid fragment can be caused. The homology between sequence C or D and base sequence of the above nucleic acid fragment 5' end or 3' end in a range possible to cause homologous recombination is, for example, 70% or more, 80% or more, 90% or more, 95% or more, 96% or more, 97% or more, 98% or more, 99% or more, 100%.

The retroviral vectors including the lentiviral vector have two sequences of 5' LTR and 3' LTR as shown in FIG. 1. For example, the base sequence of 5' LTR is as follows;

For example, the base sequence of 3' LTR is as follows; Therefore, these two sequences are homologous.

Marker gene X is included in a vector of this disclosure because marker gene X is a marker for identification of a host which expresses a gene containing in a vector of this disclosure. Actual function and action of marker gene X is described hereinafter.

As is described in the above, marker gene X is a gene to provide a marker for identification of a host which expresses gene containing vector of this disclosure and is not limited in particular as long as it is a gene which can provide a marker having such function. Marker gene X is, for example, fluorescence protein gene, drug-resistant gene, reporter gene, etc.

Fluorescence protein gene is a gene encoding fluorescence protein such as GFP, YFP, CFP, BFP, Venus, DsRed, HcRed, AsRed, ZsGreen, ZsYellow, AmCyan, AcGFP and Kaede.

Drug-resistant genes are various drug-resistant genes such as ampicillin resistant gene, kanamycin/neomycin resistance gene, tetracycline resistant gene and chloramphenicol resistant gene.

Reporter genes include such as luciferase gene and β-galactosidase.

In the vector of this disclosure, it is also possible that replication origin and sequence A are indirectly adjacent to one another and marker gene Y different from above marker gene X is contained between the above adjacent replication origin and sequence A. The above example of marker gene X is one of example of the above marker gene. Marker gene Y is not a marker which is directly related to function for selection of vector in which a foreign gene has been introduced of vector of this disclosure by homologous recombination but a marker gene which is originally contained in the above conventional retroviral vector and lentiviral vector. This means coexistence of such marker gene does not exclude in this disclosure. That is to say, the function of vector of this disclosure is not interfered even if a gene different from such marker gene exists at the above site.

The important matter with respect to a vector of this disclosure is that marker gene X is located between sequence A and sequences C and D. Such position makes it possible to selectively sort only a host containing vector where foreign gene is introduced between sequences C and D by homologous recombination. On the other hand, since above marker gene Y exists between replication origin and sequence A, such marker gene Y is contained not only in a vector in which a foreign gene has been introduced between sequences C and D after homologous recombination but also in a host containing a vector not introduced foreign gene because of homologous recombination caused between sequences A and B. Therefore, such marker gene Y is not able to use for the above selective sorting. This point is explained below further in detail.

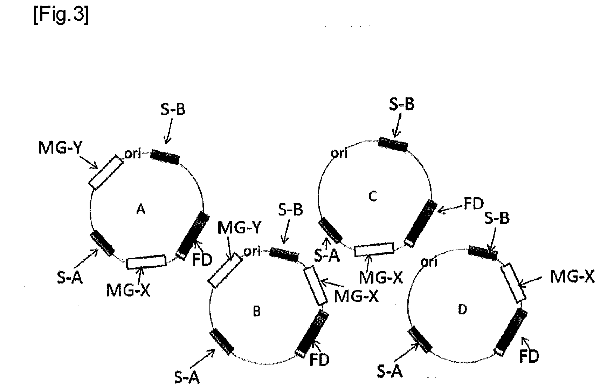

FIG. 3 shows four examples of vector of this disclosure into which a foreign gene (desired DNA) is introduced. In FIG. 3, marker gene Y (selective marker Y) is located adjacent to DNA replication origin (ori). Marker gene X (selective marker X) can be located on either side of desired gene (foreign gene) to be introduced. Preferable side is decided according to the purpose. A of vector in FIG. 3 is a vector in which a foreign gene has been introduced (desired DNA) by homologous recombination into a vector containing replication origin, marker gene Y (selective marker Y), sequence A, marker gene X (selective marker X), 2 sequences C and D for introducing foreign gene (desired DNA) by homologous recombination, and sequence B homologous with the above sequence A in that order. B of vector in FIG. 3 is a vector in which a foreign gene has been introduced (desired DNA) by homologous recombination into a vector containing replication origin, marker gene Y (selective marker Y), sequence A, 2 sequences C and D for introducing foreign gene (desired DNA) by homologous recombination, marker gene X (selective marker X), and sequence B homologous with the above sequence A in that order. C of vector in FIG. 3 is a vector in which a foreign gene has been introduced (desired DNA) by homologous recombination into a vector containing replication origin, sequence A, marker gene X (selective marker X), 2 sequences C and D for introducing foreign gene (desired DNA) by homologous recombination, and sequence B homologous with the above sequence A in that order. D of vector in FIG. 3 is a vector in which a foreign gene has been introduced (desired DNA) by homologous recombination into a vector containing replication origin, sequence A, 2 sequences C and D for introduction foreign gene (desired DNA), marker gene X (selective marker X), and sequence B homologous with the above sequence A in that order.

For example, resistant marker Y in A of FIG. 2 is ampicillin-resistant gene. When desired gene is tried to introduce into this vector using homologous recombination, it is supposed to obtain a vector, ideally like a vector shown at the bottom portion of A in FIG. 2, inserted a desired gene to a target site. However, since the above homologous sequence exists in a retroviral vector, homologous recombination cause not between desired gene and retroviral vector but in homologous sequences within retroviral vector with more high frequency as is shown in FIG. 2B, and by-product, as is shown at the bottom portion of B in FIG. 2, which is different from desired vector and original vector is produced.

In order to solve the above problem with conventional vector, in the vector of this disclosure, marker gene X (selective marker X) is arranged between indirectly adjacent the above sequence A and sequence C for introducing foreign gene by homologous recombination (see C and D of FIG. 2). In case that a foreign gene is introduced into a vector of this disclosure by homologous recombination, it is supposed that the by-product produced by homologous recombination between homologous sequence A and sequence B each other within vector in this disclosure is a product 1) contains replication origin and does not contain marker gene X and 2) contains marker gene X and does not contain replication origin. Since a by-product containing marker gene X and not containing replication origin does not contain replication origin, such by-product is never replicated within the host. On the other hand, a by-product containing replication origin and not containing marker gene X is replicated within the host, but a marker gene never express. Therefore, in use of vector of this invention, if transformant expressing marker gene is selected basing on expression or non-expression of marker gene as the index, it is possible to exclude transformant introduced the above by-product and to efficiently select a vector in which a foreign gene has been introduced (desired gene).

### (Preparation method in this invention)

This invention furthermore includes the following processes for method to prepare a vector in which a foreign gene has been introduced between the above adjacent sequences C and D, in the above vectors of this invention according to claim 1;
Step (1) for preparation of vectors in this invention,
Step (2) for preparation of nucleic acid containing sequence C' homologous with sequence C, foreign gene, and sequence D, homologous with the above sequence D, in that order,
Step (3) for production of vector according to claim 1 which foreign genes was introduced between the above adjacent sequence C and D and for formation of hosts containing such vector by exposing the above vector and the above nucleic acid fragment of Step (2) under conditions causing homologous recombination,
Step (4) for cultivation of host obtained from Step (3), and selection of a host containing vector according to claim 1 in which a foreign gene has been introduced between sequences C and D, what is chosen by expression of marker gene X from culture as index, and
Step (5) for extraction of vector in which a foreign gene has been introduced between sequences C and D from host obtained by Step (3).

In Step (1), the above vector of this invention is prepared. In a vector containing two sequences homologous with one another, sequences for insertion of desired gene between such homologous sequences by homologous recombination and homologous recombination marker gene is inserted to upstream or downstream site of such sequence by genetic engineering method like ligation, etc.

For example, using genetic engineering method, the above vector of this invention can be prepared by insertion of homologous recombination marker gene X into conventional retroviral vector (including the lentiviral vector) between 5' LTR or 3' LTR and sequence (sequence C or D) for insertion of foreign gene by homologous recombination, wherein such LTR and sequence are adjacent to each other. In this case, sequences C and D for insertion of foreign gene by homologous recombination may be conventional sequence existing in retroviral vector or be sequence newly introduced into such retroviral vector.

Step (2) is a process for preparation of insert (inset) containing foreign gene to be introduced by homologous recombination into vector of this invention. In this Step (2), nucleic acid fragment containing sequence C' homologous with sequence C existing in vector of this invention, foreign gene and sequence D' homologous with sequence D existing in vector of this invention in that order is prepared. Such nucleic acid fragment can be prepared, by use of 1) forward primer containing sequence C' in 5' end region and 2) reverse primer containing sequence D' on 5' end region, with nucleic acid amplification method such as PCR method using nucleic acid fragment including foreign gene as genetic template.

Step (3) is a process for 1) creation of vector in which a foreign gene has been introduced between adjacent sequences C and D and 2) formation of host containing vector in which such foreign gene has been introduced under conditions causing homologous recombination between vector of this invention and nucleic acid fragment prepared by Step (2). In Step (3), homologous recombination can be performed *in vitro* and sometimes *in vivo.*

In case of *in vitro,* homologous recombination between vector of this invention and the above nucleic acid fragment can be performed, for example, by 1) *in vitro* mixing of enzyme to cause homologous recombination, vector of this invention and the above nucleic acid fragment and then 2) incubation of such mixture for appropriate time and at appropriate temperature. Enzymes causing homologous recombination include various recombinase, and commercialized products of cloning system using such recombinase include In-Fusion Advantage PCR cloning kit (Clontech Laboratories, Inc.), Gateway system (Invitrogen Japan K.K.). Then, host able to replicate vector of this invention is transformed using reactant of such homologous recombination so as to form the host containing vector in which a foreign gene has been introduced. As the host to be used, it is only necessary to be suitable for replication origin in vector of this invention; for example, Escherichia coli, bacteriophage, yeast (e.g., Saccharomyces cerevisiae, Schizosaccharomyces pombe). As transformation method, various methods in the public domain can be used according to the kind of host.

In case of *in vivo* alternative to the above *in vitro* homologous recombination method, Step (3) can be performed by 1) obtaining a) gene encoding enzyme for homologous recombination and b) host containing vector of this invention and nucleic acid fragment prepared in Step (2), and then 2) expressing gene encoding enzyme for the above homologous recombination in the obtained host. For example, foreign gene can be introduced into vector of this invention by 1) transformation of vector of this invention and nucleic acid fragment prepared in Step (2) in host containing gene encoding enzyme for homologous recombination, 2) expression of gene encoding enzyme for homologous recombination in host, and 3) homologous recombination in host. In this case, because of expression of enzyme to cause homologous recombination in a host, the vector of this invention and the above nucleic acid fragment cause homologous recombination by activity of such enzyme in the host, and then a host containing vector in which a foreign gene has been introduced is obtained. As enzyme for homologous recombination, pRedET contained in kit of Red/ET homologous recombination sold by Gene Bridges GmbH is included. A cell introduced this into a host Escherichia coli (DH10B, HS996, Gene Hogs or TOP10) is used as a competent cell.

GenLantis sells Xi-Clone High Speed Cloning Kit utilizing original property of a host Escherichia coli with respect to homologous recombination without introducing a gene encoding enzyme for homologous recombination.

Step (4) is a process for selection of a host containing vector in which a foreign gene has been introduced between sequences C and D in vector of this invention by 1) cultivating the host (transformant) obtained in Step (3) and 2) selecting a host expressing a marker gene X from culture. In case that a marker gene is fluorescence protein gene, a host expressing such fluorescence protein can be selected by fluorescence detection. In case that a marker gene X is drug-resistant gene, only a host expressing drug-resistant gene (marker gene X) can be selected if the host (transformant) obtained from medium containing corresponding drug in Step (3) is cultivated. For example, in case that Escherichia coli is used as a host and ampicillin-resistant gene is used as a marker gene X, only Escherichia coli expressing ampicillin-resistant gene can be selected by cultivating Escherichia coli transformant obtained on a plate medium containing ampicillin in Step (3).

As is described in the above, a by-product produced by causing homologous recombination between sequence A and sequence B homologous with one another in vector of this invention is supposed 1) to contain a replication origin and not to contain a marker gene X and 2) to contain a marker gene X and not to contain a replication origin. The by-product which contains a marker gene X and does not contain a replication origin is not replicated in the host because of lack of a replication origin. On the other hand, with respect to a by-product which contains a replication origin and does not contain a marker gene X, marker gene X is not expressed in the host. For example, in case that Escherichia coli is used as a host and ampicillin-resistant gene is used as a marker gene X, if Escherichia coli transformant obtained in Step (3) is cultivated on a plate medium containing ampicillin, Escherichia coli introduced a by-product containing ampicillin-resistant gene (marker gene X) and not containing a replication origin has such by-product lacking the replication origin, and such by-product is not replicated in Escherichia coli host and ampicillin-resistant gene is not expressed. Therefore, Escherichia coli introduced a by-product containing ampicillin-resistant gene (marker gene X) and not containing the replication origin is not able to grow on a plate medium containing ampicillin and to form colony. On the other hand, Escherichia coli introduced by-product containing a replication origin and not containing ampicillin-resistant gene (marker gene X) has such by-product containing replication origin, and such by-product is possible to be replicated in Escherichia coli host and ampicillin-resistant gene is not expressed in such Escherichia coli host. Therefore, Escherichia coli introduced a by-product containing a replication origin and not containing ampicillin-resistant gene (marker gene X) is not able to grow on a plate medium containing ampicillin and to form colony. In this way by this invention, the host introduced the above by-product is excluded basing on expression or non-expression of marker gene X as the index, and as the result, it is possible to efficiently select a host containing a vector in which a foreign gene has been introduced by homologous recombination in a vector of this invention.

Step (5) is a process to extract a vector in which a foreign gene has been introduced between sequences C and D in vector of this invention, from the host selected in Step (4).

As a method for extraction of a vector in which a foreign gene has been introduced between sequences C and D in a vector of this invention, various publicly known nucleic acid extraction technology are included. For example, in case that the host is Escherichia coli, it is possible to extract a vector in which a foreign gene has been introduced between sequences C and D in a vector of this invention, from Escherichia coli host by various publicly known plasmid extraction method.

This invention is specifically explained by showing examples in the followings, but this invention is not limited to the following examples.

### EXAMPLES

### (comparative examples)

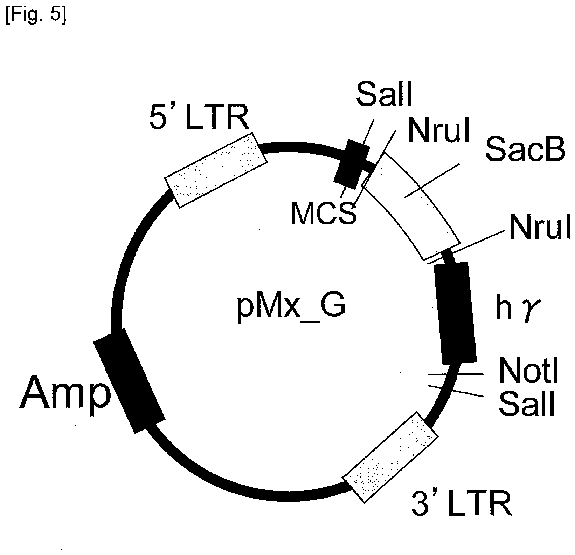

An antibody gene is intended to be introduced into a conventional retroviral vector pMX_G (FIG. 5) having a structure as shown in FIG. 2A by homologous recombination method of Gene Bridges GmbH or Clontech Laboratories, Inc. (Takara Bio Inc.). The meanings of code symbol in FIG. 5 are as follows;
pMx_G : Vector for expression of antibody H chain
Amp : Ampicillin-resistant gene
hy: Antibody heavy chain constant region cDNA
SacB : Bacillus subtilis genomic DNA
   Escherichia coli is not able to survive in sucrose medium if such gene exists.
LTR : Long Terminal Repeat
MCS : Multi-cloning site
NruI, NotI, SalI : Restriction enzyme sites

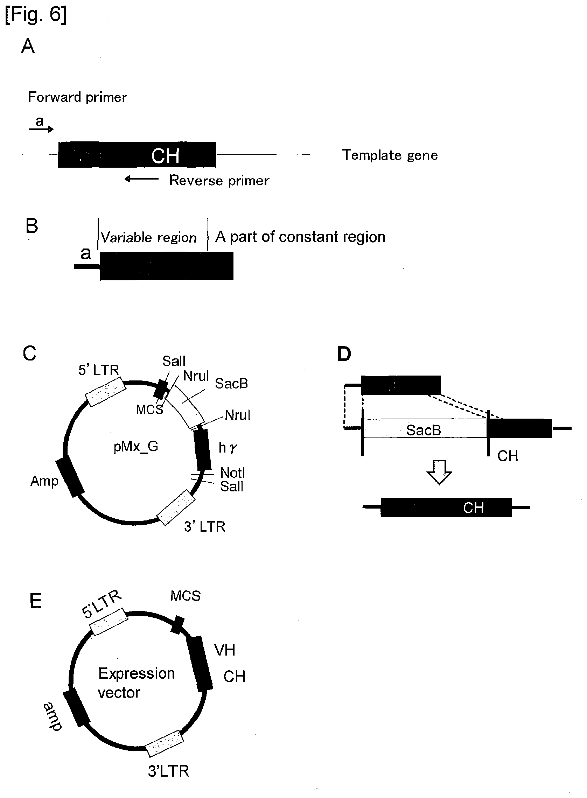

At first, as shown in FIG. 6A, from a template DNA containing a gene of antibody heavy chain, a gene is amplified by PCR using 1) a forward primer containing sequence of "a" (approximately 20 bases) at 5' end and 2) a reverse primer containing a part of constant region (CH) of an antibody (approximately 20 bases) at 5' end. As shown in FIG. 6B, the obtained PCR product is a product added 1) sequence of "a" at the end of antibody variable region (VH) and 2) a part of constant region of antibody heavy chain. Upstream 20 bases of NruI site located at upstream of SacB in retroviral vector shown in FIG. 6C is identical to the sequence of "a" at end of PCR product of FIG. 6B. Furthermore, some sequence of heavy chain constant region of downstream of Nrul site located at downstream of SacB gene is the same sequence with some heavy chain constant region at the end of PCR product of FIG. 6B (NruI site is not contained in the sequence of PCR product of FIG. 6B). When the vector of FIG. 6C received NruI treatment is introduced together with PCR product of FIG. 6B into homologous recombination Escherichia coli, it is expected to cause homologous recombination like FIG. 6D and to obtain an expression vector having a structure of FIG. 6E. However, in fact probability to obtain the expected vector was low because homologous recombination occurs at high rates. The meanings of code symbols in FIG. 6 are as follows;
pMx_G: Vector for antibody heavy chain expression
Amp: Ampicillin-resistant gene
hγ: Antibody heavy chain constant region cDNA
SacB: Bacillus subtilis genome derived DNA
   Escherichia coli is not able to survive in sucrose medium if such gene exists.
LTR: Long Terminal Repeat
SalI, NruI, NotI: Restriction enzyme sites

Detail of each procedure is explained below.

### 〈Introducing human antibody heavy chain gene into retroviral vector by homologous recombination〉

### [Cut of vector by restriction enzyme (linearization)]

Two µg of vectors was cut at 37°C for 2 hours using NruI restriction enzyme (10 U) (Roche Diagnostic K.K.) under existence of Buffer B (Roche Diagnostic K.K.) in 50µL of reaction solution and was linearized. After treatment with restriction enzyme, 1µL of the reaction solution was electrophoresed with 0.6% of agarose gel at 100V for 20 minutes and vector 6kb and SavB 1.9kb bands were confirmed. Remaining 49µL of reaction solution was purified with QIAquick PCR Purification kit (Qiagen N.V.) and was eluted with 30µL of EB solution attached to the kit.

### [Preparation of insert DNA]

Sequence possible to insert by homologous recombination into a vector is added to an insert DNA to be introduced into the vector, by amplification of a desired insert (antibody heavy chain) with PCR using 1) a forward primer containing 20 bases homologous with multi-cloning site (MCS) of 5' upstream of Nrul site on 5' side of vector and 2) a reverse primer containing homologous 20 bases at heavy chain constant region of Nrul site of 3' downstream on 3' side of vector. (FIG. 6A and B)

### [Gene introduction (Gene Bridges GmbH) to Escherichia coli]

One µL of vector and 1µL of insert DNA were mixed in 1.5mL of microtube, and 50µL of Escherichia coli competent cells (Gene Bridges GmbH) for homologous recombination was further added and mixed on ice, and then the mixture was allowed to stand on ice for 15 minutes. After heat-shock for 30 seconds at 42°C, the mixture was further allowed to stand on ice for 5 minutes. And then, after the microtube was incubated for 1 hour at 37°C, the solution in the microtube was plated to LB (Luria-Bertani) agar medium (10 cm dish) containing 100µg/mL ampicillin, 2% sucrose (Nacalai Tesque, Inc.). Standing culture of this plate was performed for 14 hours at37°C. The composition of used LB agar medium was as follows;
1% polypeptone (Wako Pure Chemical Industries, Ltd.)
0.5% dried yeast extract (Nacalai Tesque, Inc.)
1% sodium chloride (Wako Pure Chemical Industries, Ltd.)
1% agar powder (Nacalai Tesque, Inc.)
*The following reagents were contained in selection medium.
2% sucrose (Nacalai Tesque, Inc.)
100µg/mL ampicillin (Wako Pure Chemical Industries, Ltd.)

### [Homologous recombination and introducing gene into Escherichia coli with the use of In-fusion Advantage PCR cloning kit (Clontech Laboratories, Inc.: Takara Bio Inc.)]

5 µL of PCR insert and 2µL of Cloning Enhancer attached to the kit were mixed in 1.5mL of microtube. After incubation for 15 minutes at 37°C, the mixture was incubated for 15 minute at 80°C and was allowed to stand on ice. 7 µL of linearized vector and 1µL of the above PCR insert were mixed with 1µL of In-fusion enzyme and 2µL of 5×In-fusion reaction buffer attached to the kit in 1.5mL of microtube. After incubation for 15 minutes at 37°C, the mixture was incubated for 15 minute at 50°C and was allowed to stand on ice. Thereafter, TE buffer was added so as to be 50µL in total volume. 2.5µL of this mixture and 50µL of Escherichia coli (DH5α) competent cells were added and mixed in 1.5µL of microtube, and were allowed to stand for 15 minutes on ice. After heat shock for 30 seconds at 42°C, the mixture was allowed to stand for another 5 minutes on ice. And then, after the microtube was incubated for 1 hour at 37°C, the solution in the microtube was plated with 100µg/mL ampicillin, LB (Luria-Bertani) agar medium (a 10cm dish) containing 2% sucrose (Nacalai Tesque, Inc.). Standing culture of this plate was performed for 14 hours at 37°C. Composition of the used LB agar medium was as follows;
1% polypeptone (Wako Pure Chemical Industries, Ltd.)
0.5% dried yeast extract (Nacalai Tesque, Inc.)
1% sodium chloride (Wako Pure Chemical Industries, Ltd.)
1% agar powder (Nacalai Tesque, Inc.)
*The following reagents were contained in selection medium.
2% sucrose (Nacalai Tesque, Inc.)
100µg/mL ampicillin (Wako Pure Chemical Industries, Ltd.)

### [Culture of Escherichia coli from Escherichia coli single colony]

7-9 colonies of Escherichia coli were picked up and shaking culture of each colony was performed for 14 hours at 37°C in 2mL of liquid LB medium containing 100µg/mL ampicillin.

### [DNA purification]

Plasmid DNA was extracted using QIAprep Miniprep kit (Qiagen N.V.) from the above Escherichia coli and was eluted using 50µL of the attached EB solution.

### [Insert check by restriction enzyme SalI (per 1 sample)]

0.2 µg of vector was received restriction enzyme treatment for 2 hours at 37°C by SalI (15 IU) (Nippon Gene Co., Ltd) in the presence of Buffer H (Nippon Gene Co., Ltd.) in reaction liquid (total volume: 10.1µL). Thereafter, the reaction solution (10µL) was electrophoresed for 20 minutes at 100V with 0.6% of agarose gels.

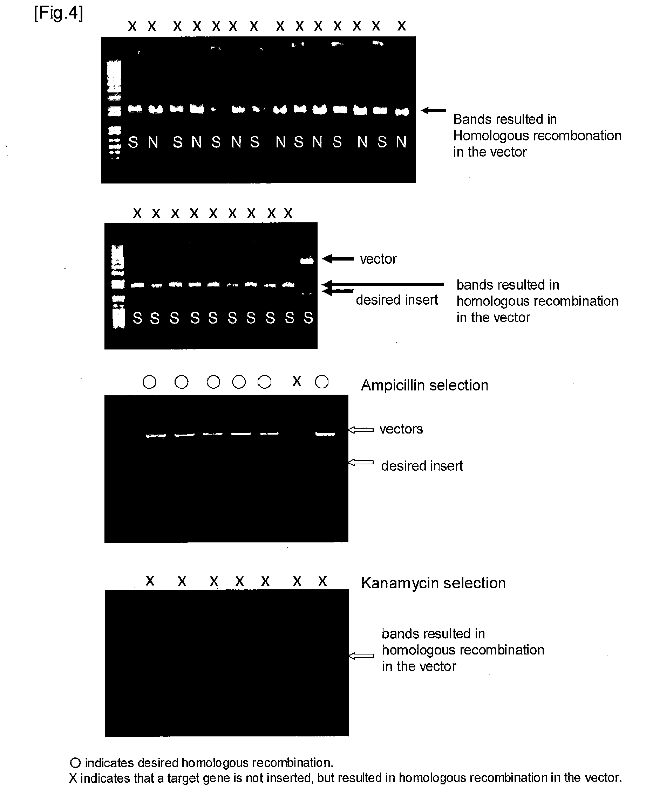

Two bands of desired gene (about 1kbp from vector) should be observed at about 1kbp from vector like right-side of FIG. 4B according to the expectation, but the size of the vector was small in comparison with the original vector because homologous recombination was caused (as is FIG. 2B) between 5'-LTR and 3'-LTR of the used retroviral vector and DNA was not cut by the used restriction enzyme SalI (FIG. 4A, B).

### (Example 1)

FIG. 4C shows 2 examples of selection by ampicillin only and kanamycin only, respectively after introducing TCR gene by homologous recombination using a retroviral vector prepared according to this invention. The detailed explanation is as follows;

### [Introducing mouse T-cell receptor (TCR) α-chain into a vector of this invention by homologous recombination: in case of selection by ampicillin alone and kanamaycin alone]

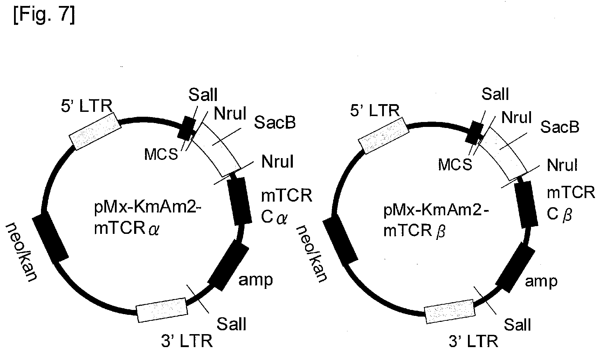

Retroviral vector pMX-KmAm2-mα for homologous recombination shown in left side of FIG. 7 was a vector inserted sequence (MCS, NruI, SacB, NruI, a part of mouse TCRα constant region) for insertion of a desired gene (variable region of mouse TCRα) by homologous recombination after substitution of ampicillin-resistant gene of pMX vector (publicly known retroviral vector) with kanamycin-resistant gene by genetic engineering method, and was inserted ampicillin-resistant gene between mouse TCRα and 3 'LTR as a vector modification concerning this invention. Cloning was performed by homologous recombination of mouse TCRα chain using retroviral vector pMX-KmAm2-mα (containing constant region of mouse TCRα and being introduced ampicillin-resistant gene into downstream of 5 'LTR). The code symbols in FIG. have the following means;
pMx-KmAm2-mTCRα: Vector for expressing TCRα chain
pMx-KmAm2-mTCRβ: Vector for expressing TCRβ chain
Neo/kan: neomycin/kanamycin-resistance gene
Amp: Ampicillin-resistant gene
mTCR Cα: TCR α-chain constant region cDNA
mTCR Cβ: TCR β-chain constant region cDNA
SacB: Bacillus subtilis genome derived DNA
   If this gene exists, Escherichia coli is not able to survive in sucrose medium.
LTR: Long Terminal Repeat
SalI, NruI: Restriction enzyme sites.

In retroviral vector pMX-KmAm2-mα for homologous recombination, a gene corresponding to resistant marker Y (marker gene Y) in the vector shown in FIG. 2 is kanamycin-resistant gene, and a gene corresponding to resistant marker X (marker gene X) is ampicillin-resistant gene. In case that only kanamycin of resistant marker Y is selected, Escherichia coli into which a vector, out of vectors caused homologous recombination between 5 'LTR and 3' LTR, having kanamycin-resistance gene is introduced also grows out (FIG. 2B). On the other hand, in case that only ampicillin of resistant marker X is selected, because a vector, out of vectors caused homologous recombination between 5 'LTR and 3' LTR, having ampicillin-resistant gene has no DNA replication origin, Escherichia coli into which such gene is introduced is not able to grow. Because a vector, out of vectors caused homologous recombination between 5 'LTR and 3' LTR, having DNA replication origin has no ampicillin-resistant gene, Escherichia coli into which such gene is introduced is not also able to grow. From these findings, it is presumed that Escherichia coli introduced desired gene is selected.

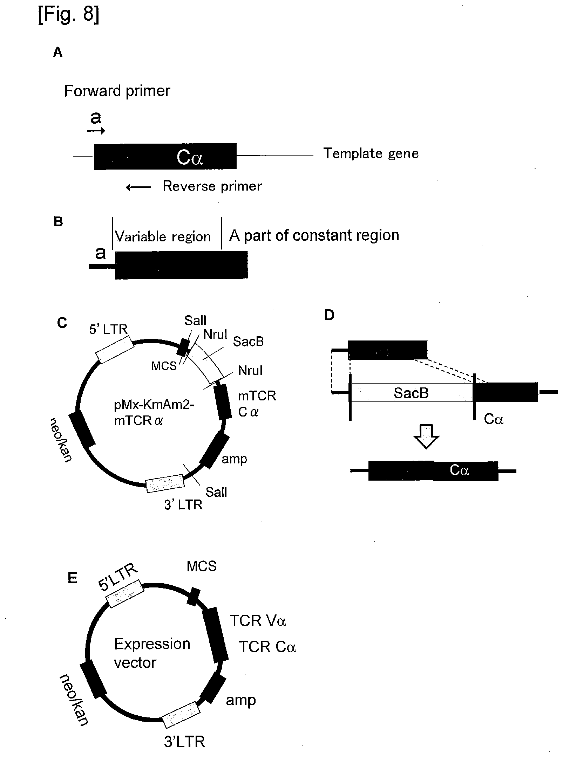

At first, as shown in FIG. 8A, a gene from template DNA containing TCR gene is amplified by PCR using reverse primer containing a part of constant region of TCR (approximately 20 bases) at forward primer 5' end containing sequence of "a" (approximately 20 bases) at 5' end. As shown in FIG. 8B, the obtained PCR product is a product added sequence of "a" and a part of constant region of TCR at the end. In a cloning vector with a structure of this invention shown in FIG. 8C, upstream 20 bases of NruI site located on upstream of SacB gene are identical to sequence of "a" of PCR product's end in FIG. 8B. Some sequence in mouse TCRα constant region at downstream of NruI site located on downstream of SacB gene is the same sequence with some constant region of TCR of the PCR product of FIG. 8B. (NruI site is not contained in sequence of FIG. 8B). When NruI treatment of vector of FIG. 8C is performed and Escherichia coli for homologous recombination (TOP10 introduced plasmid pREDET [Gene Bridges GmbH]) is introduced together with PCR product of FIG. 8B, homologous recombination like FIG. 8E is caused and the expression vector with structure of FIG. 8E is obtained. The meanings of code symbols in FIG. 8 are as follows;
pMx-KmAm2-mTCRα: Vector for expressing TCRα chain
pMx-KmAm2-mTCRβ: Vector for expressing TCRβ chain
Neo/kan: Neomycin/kanamycin-resistance gene
Amp: Ampicillin-resistant gene
mTCR Cα: TCRα chain constant region cDNA
mTCR Cβ: TCRβ chain constant region cDNA
SacB: Bacillus subtilis genomic DNA
   Escherichia coli is not able to survive in sucrose medium in case of existence of
   this gene.
LTR: Long Terminal Repeat
Detail of each protocol is explained below.

### [Cutting (linearization) of vector by restriction enzyme]

Two µg of vector was cut for 2 hours at 37°C in 50µL of reaction solution in the presence of Buffer B (Roche Diagnostic K.K.) using NruI restriction enzyme (10 U) (Roche) and was linearized. Upon restriction enzyme treatment, 1µL of reaction solution was electrophoresed with 0.6% of agarose gels for 20 minutes at 100V and bands of vector 6kb and SacB 1.9kb were confirmed. Remaining 49µL was purified with QI Aquick PCR Purification kit (Qiagen N.V.) and was eluted with 30µL of EB solution attached to the kit.

### [Preparation of insert DNA]

Insertable sequence is added into an insert DNA to be introduced to a vector by amplification of a desired insert (TCR) by PCR using 1) a forward primer containing sequence of 20 bases homologous with multi-cloning site (MCS) of 5' upstream at NruI site of vector 5' side and 2) a reverse primer containing sequence of 20 bases homologous with TCR constant region of 3' downstream at NruI site of vector 3' side (FIG. 8).

### [Gene introduction into Escherichia coli (Gene Bridges GmbH)]

One µL of a vector and 1µL of an insert DNA was mixed in 1.5 mL of microtube, and 50µL of Escherichia coli competent cell for homologous recombination was further added into and mixed in the microtube on ice. The microtube was allowed to stand on ice for 15 minutes. After heat shock for 30 seconds at 42°C, the microtube was further allowed to stand for 5 minutes on ice. Thereafter, the microtube was incubated for 1 hour at 37°C and then the solution in the microtube was plated to 1) LB (Luria-Bertani) agar medium (10cm dish) containing 100µg/mL ampicillin and 2% sucrose (Nacalai Tesque, Inc.) or 2) LB agar medium containing 50µg/mL kanamycin and 2% sucrose. Standing culture of this plate was performed for 14 hours at 37°C. The composition of the used LB agar medium was as follows;
1% polypeptone (Wako Pure Chemical Industries, Ltd.)
0.5% dried yeast extract (Nacalai Tesque, Inc.)
1% sodium chloride (Wako Pure Chemical Industries, Ltd.)
1% agar powder (Nacalai Tesque, Inc.)
* The following reagents are contained as a medium for selection.
2% sucrose (Nacalai Tesque, Inc.)
100µg/mL ampicillin (Wako Pure Chemical Industries, Ltd.) or 50µg/mL kanamycin sulfate injection (Meiji Seika Pharma Co., Ltd.)

### [Culture of Escherichia coli from Escherichia coli single colony]

Twelve colonies of Escherichia coli were picked up and shaking culture of each colony was performed for 14 hours at 37°C in 2mL of liquid LB medium containing 100µg/mL ampicillin or 50µg/mL kanamycin.

### [DNA purification]

Plasmid DNA was extracted from the above Escherichia coli using QIAprep Miniprep kit (Qiagen N.V.) and was eluted using 50µL of the attached EB solution.

### [Insert check by restriction enzyme SalI (per 1 sample)]

Restriction enzyme treatment of 0.2µg of vector with SalI (15U) (Nippon Gene Co., Ltd.) was performed for 2 hours at 37°C in 10.1µL (total volume) of reaction solution in the presence of Buffer H (Nippon Gene Co., Ltd.). Thereafter, the reaction solution (10µL) was electrophoresed with 0.6% agarose gel for 20 minutes at 100V, and a vector band 4.5kb and a desired DNA OT1 TCRα+Amp band 1.9kb were confirmed (FIG. 4C). As the result, as shown in FIG. 4C, in case of selection by ampicillin only, it was confirmed that the desired gene was exactly introduced by homologous recombination to the desired site in 6 plasmid DNAs out of 7 plasmid DNAs. In case of selection by kanamycin only, it was confirmed that no desired gene was introduced by homologous recombination to the desired site in any of 7 plasmid DNAs.

### (Example 2)

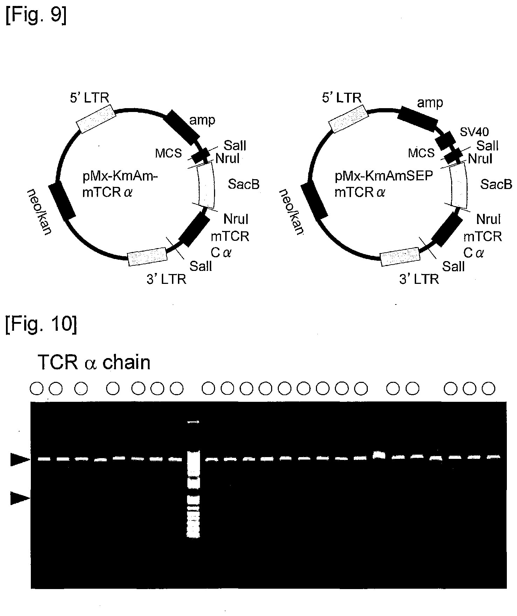

As the other example of vector in this invention, 2 kinds of vectors shown in FIG. 9 were prepared. Retroviral vector pMX-KmAm-mα for homology recombination shown in the FIG. 9 left is a vector inserted sequence (MCS, NruI, SacB, Nrul, a part of mouse TCRα constant region) for insertion of desired gene (variable region of mouse TCRα) by homology recombination from way of substitution of ampicillin-resistant gene of pMX vector (publicly known retroviral vector) to kanamycin-resistant gene by genetic engineering method. As a modification of vector in this invention, ampicillin-resistant gene is inserted between 5 'LTR and mouse TCRα. In this vector, the locational relation between ampicillin-resistant gene (amp) and desired gene is different from pMx-KmAm2-mTCRα shown in FIG. 5, but this vector is able to use for selection of intended homology recombination.

In pMx-KmAmSEP-mTCRα shown in the right of FIG. 9, the location of ampicillin-resistant gene (amp) is the same with pMx-KmAm-mTCRα shown in the left of FIG. 9; near to 5 'LTR. Furthermore, SV40 enhancer and promoter (SV40) are inserted to the foreside of TCR gene in order to enhance the expression of TCR gene. The meaning of each code symbol in FIG. 9 is as follows;
Neo/kan: Neomycin/kanamycin-resistance gene
Amp: Ampicillin-resistant gene
mTCR Cα: TCRα chain constant region cDNA
mTCR Cβ: TCRβ chain constant region cDNA
SacB: Bacillus subtilis genome derived DNA

### (Example 3)

### [Cloning of functional TCR gene from human T lymphocyte]

Cloning of a desired DNA was performed actually using a vector of this invention for insertion of a desired DNA fragment by homologous recombination method. The detailed procedure was as follows;
Human T lymphocyte was sorted by a cell sorter so as to be one cell in each well of 96well PCR plate.

TCR Vα region and TCR Vβ region were amplified by 5'-RACE method from near site of constant region 5' of TCR to upstream of TCR V gene. The amplified TCR Vα region and TCR Vβ region were incorporated using homologous recombination system of Gene Bridges GmbH or Clontech Laboratories, Inc. into an expression vector for homologous recombination; pMX-KmAm2-hTCRα, pMX-KmAm2-hTCRβ [vector where ampicillin-resistant gene of pMX vector which is a publicly known retroviral vector is substituted to kanamycin-resistant gene by genetic engineering method in advance and sequences (MCS, NruI, SacB, NruI, a part of human TCR gene constant region) for insertion are inserted.].

A vector obtained by conventional method was introduced into Escherichia coli and Escherichia coli resistant both to ampicillin and kanamycin were cultured overnight on agar medium, and the expressed colonies were picked up one by one and were cultured in LB medium containing 2mL of ampicillin and kanamycin. On the next day, plasmid DNA was purified, was cut with both restriction enzymes BamHI and NotI and was analyzed with agarose gel.

As the result, an intended band was detected with respect to 21 out of 24 plasmid DNA as shown in the following FIG. 10 (Result on TCR Vα. The upper bands are the band of vector and the lower bands are the band of TCR α chain cDNA).

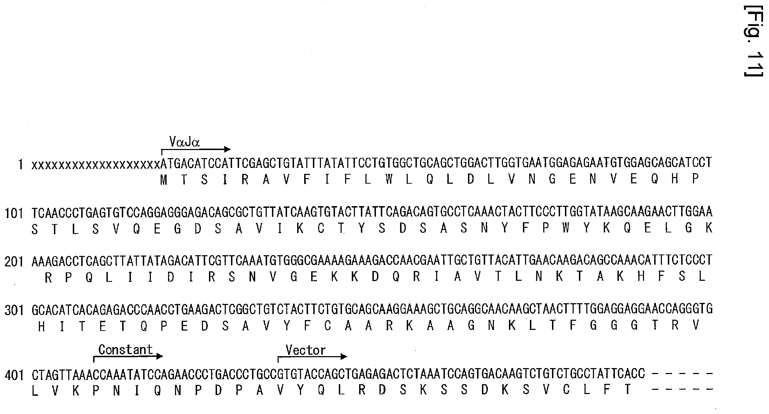

Then, when the sequence of insert of plasmid DNA was analyzed, a functional TCR gene was confirmed to be inserted into the expression vector in frame as shown in FIG. 11 and sequences No. 3 and 4 of sequence listing.

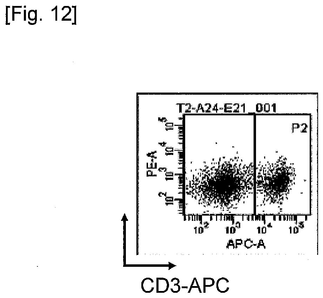

The obtained TCRα chain and TCRβ chain genes were simultaneously introduced into mouse T cell strain TG40 which does not express endogenous TCR gene, and the expression of mouse CD3 molecule to cellular surface was analyzed. As the result, expression of CD3 molecule to cellular surface was confirmed by flow cytometry analysis (FIG. 12). This result shows that the obtained TCR gene is a functional TCR gene because CD3 molecule was expressed on cellular surface only in the form with TCR.

## Claims

1. A method of producing a recombinant retroviral vector in which a foreign gene has been introduced, comprising the steps of;
(1) preparing a retroviral vector comprising a replication origin, sequence A, marker gene X, sequences C and D for introducing a foreign gene by homologous recombination, and sequence B that is homologous with said sequence A, in that order, wherein said sequences C and D are directly or indirectly adjacent, and for use in introducing the foreign gene between said adjacent sequences C and D;
(2) preparing a nucleic acid fragment containing sequence C' that is homologous with said sequence C, a foreign gene, and sequence D' that is homologous with said sequence D, in that order;
(3) by exposing said retroviral vector and said nucleic acid fragment to conditions to cause homologous recombination, generating a recombinant retroviral vector in which the foreign gene has been introduced between said adjacent sequences C and D, and forming a host that contains the recombinant retroviral vector in which the foreign gene has been introduced between said adjacent sequences C and D;
(4) culturing the host obtained in Step (3), and selecting, from the culture, a host containing the recombinant retroviral vector in which the foreign gene has been introduced between said adjacent sequences C and D by identifying the expression of marker gene X as an indicator; and
(5) extracting from the host selected in Step (4) the recombinant retroviral vector in which the foreign gene has been introduced between said adjacent sequences C and D,
wherein said sequence A is 3 'LTR contained in a retroviral genome and said sequence B is 5' LTR contained in a retroviral genome, or said sequence A is 5' LTR contained in a retroviral genome and said sequence B is 3' LTR contained in a retroviral genome.

2. The method according to Claim 1 wherein said Step (3) is performed by mixing said retroviral vector and said nucleic acid fragment with an enzyme for homologous recombination to obtain a homologous recombination product, and transforming a host with the obtained product.

3. The method according to Claim 1, wherein said Step (3) is performed by obtaining a gene encoding an enzyme for homologous recombination and a host containing said nucleic acid fragment and said retroviral vector, and expressing the gene encoding an enzyme for homologous recombination in the obtained host.

4. The method according to Claim 1 to 3, wherein the retroviral vector is a lentiviral vector.

5. The method according to any one of Claims 1 to 4, wherein marker gene X is a fluorescence protein gene or a drug-resistant gene.

6. The method according to any one of Claims 1 to 4, wherein marker gene X is a gene encoding luciferase or β-galactosidase.

7. The method according to any one of Claims 1 to 6, wherein the replication origin and sequence A are indirectly adjacent, and marker gene Y different from marker gene X is contained between said adjacent replication origin and sequence A.

8. The method according to any one of Claims 1 to 6, wherein the replication origin and sequence B are indirectly adjacent, and marker gene Y different from marker gene X is contained between the said adjacent replication origin and sequence B.

9. The method according to Claim 7 or 8, wherein marker gene Y is a fluorescence protein gene or a drug-resistant gene.

10. The method according to Claim 7 or 8, wherein marker gene Y is a gene encoding luciferase or β-galactosidase.

11. The method according to any one of Claims 1 to 10, wherein the replication origin is a replication origin of an organism selected from Escherichia coli, bacteriophage, Saccharomyces cerevisiae and Schizosaccharomyces pombe.

12. Use of a retroviral vector comprising a replication origin, sequence A, marker gene X, sequences C and D for introducing a foreign gene by homologous recombination, and sequence B that is homologous with said sequence A, in that order, wherein said sequences C and D are directly or indirectly adjacent, and for use in introducing the foreign gene between said adjacent sequences C and D in a method according to claims 1 to 10,
wherein said sequence A is 3 'LTR contained in a retroviral genome and said sequence B is 5' LTR contained in a retroviral genome, or said sequence A is 5' LTR contained in a retroviral genome and said sequence B is 3' LTR contained in a retroviral genome.

## Patentansprüche

1. Verfahren zur Herstellung eines rekombinanten retroviralen Vektors, in den ein Fremd-Gen eingeführt wurde, umfassend die Schritte:
(1) Herstellen eines retroviralen Vektors, der einen Replikationsstartpunkt, eine Sequenz A, ein Marker-Gen X, die Sequenzen C und D zur Einführung eines Fremd-Gens durch homologe Rekombination und Sequenz B, die zu der Sequenz A homolog ist, in dieser Reihenfolge umfasst, wobei die Sequenzen C und D direkt oder indirekt benachbart sind und zur Einführung des Fremd-Gens zwischen die benachbarten Sequenzen C und D dienen;
(2) Herstellen eines Nucleinsäurefragments, das Sequenz C', die zu der Sequenz C homolog ist, ein Fremd-Gen und Sequenz D', die zu der Sequenz D homolog ist, in dieser Reihenfolge enthält;
(3) durch Einwirkenlassen von Bedingungen, die eine homologe Rekombination bewirken, auf den retroviralen Vektor und das Nucleinsäurefragment Erzeugen eines rekombinanten retroviralen Vektors, in den das Fremd-Gen zwischen den benachbarten Sequenzen C und D eingeführt wurde, und Bilden eines Wirts, der den rekombinanten retroviralen Vektor, in den das Fremd-Gen zwischen den benachbarten Sequenzen C und D eingeführt wurde, enthält;
(4) Kultivieren des in Schritt (3) erhaltenen Wirts und Auswählen eines Wirts, der den rekombinanten retroviralen Vektor, in den das Fremd-Gen zwischen den benachbarten Sequenzen C und D eingeführt wurde, enthält, aus der Kultur durch Identifizieren der Expression des Marker-Gens X als Indikator; und
(5) Extrahieren des rekombinanten retroviralen Vektors, in den das Fremd-Gen zwischen den benachbarten Sequenzen C und D eingeführt wurde, aus dem in Schritt (4) ausgewählten Wirt;
wobei die Sequenz A in einem retroviralen Genom 3'-LTR enthalten ist und die Sequenz B in einem retroviralen Genom 5'-LTR enthalten ist oder die Sequenz A in einem retroviralen Genom 5'-LTR enthalten ist und die Sequenz B in einem retroviralen Genom 3'-LTR enthalten ist.

2. Verfahren gemäß Anspruch 1, wobei Schritt (3) durchgeführt wird, indem man den retroviralen Vektor und das Nucleinsäurefragment mit einem Enzym für homologe Rekombination mischt, um ein homologes Rekombinationsprodukt zu erhalten, und einen Wirt mit dem erhaltenen Produkt transformiert.

3. Verfahren gemäß Anspruch 1, wobei Schritt (3) durchgeführt wird, indem man ein Gen, das ein Enzym für homologe Rekombination codiert, und einen Wirt, der das Nucleinsäurefragment und den retroviralen Vektor enthält, bereitstellt und das Gen, das ein Enzym für homologe Rekombination codiert, in dem bereitgestellten Wirt exprimiert.

4. Verfahren gemäß Anspruch 1 bis 3, wobei der retrovirale Vektor ein lentiviraler Vektor ist.

5. Verfahren gemäß einem der Ansprüche 1 bis 4, wobei das Marker-Gen X ein Fluoreszenzprotein-Gen oder ein Wirkstoffresistenz-Gen ist.

6. Verfahren gemäß einem der Ansprüche 1 bis 4, wobei das Marker-Gen X ein Gen ist, das Luciferase oder β-Galactosidase codiert.

7. Verfahren gemäß einem der Ansprüche 1 bis 6, wobei der Replikationsstartpunkt und Sequenz A indirekt benachbart sind und ein Marker-Gen Y, das von Marker-Gen X verschieden ist, zwischen den benachbarten Replikationsstartpunkt und Sequenz A enthalten ist.

8. Verfahren gemäß einem der Ansprüche 1 bis 6, wobei der Replikationsstartpunkt und Sequenz B indirekt benachbart sind und ein Marker-Gen Y, das von Marker-Gen X verschieden ist, zwischen den benachbarten Replikationsstartpunkt und Sequenz B enthalten ist.

9. Verfahren gemäß Anspruch 7 oder 8, wobei das Marker-Gen Y ein Fluoreszenzprotein-Gen oder ein Wirkstoffresistenz-Gen ist.

10. Verfahren gemäß Anspruch 7 oder 8, wobei das Marker-Gen Y ein Gen ist, das Luciferase oder β-Galactosidase codiert.

11. Verfahren gemäß einem der Ansprüche 1 bis 10, wobei der Replikationsstartpunkt ein Replikationsstartpunkt eines Organismus ist, der aus *Escherichia coli,* einem Bakteriophagen, *Saccharomyces cerevisiae* und *Schizosaccharomyces pombe* ausgewählt ist.

12. Verwendung eines retroviralen Vektors, der einen Replikationsstartpunkt, eine Sequenz A, ein Marker-Gen X, die Sequenzen C und D zur Einführung eines Fremd-Gens durch homologe Rekombination und Sequenz B, die zu der Sequenz A homolog ist, in dieser Reihenfolge umfasst, wobei die Sequenzen C und D direkt oder indirekt benachbart sind und zur Einführung des Fremd-Gens zwischen die benachbarten Sequenzen C und D dienen, in einem Verfahren gemäß den Ansprüchen 1 bis 10,
wobei die Sequenz A in einem retroviralen Genom 3'-LTR enthalten ist und die Sequenz B in einem retroviralen Genom 5'-LTR enthalten ist oder die Sequenz A in einem retroviralen Genom 5'-LTR enthalten ist und die Sequenz B in einem retroviralen Genom 3'-LTR enthalten ist.

## Revendications

1. Procédé de production d'un vecteur rétroviral recombinant dans lequel un gène étranger a été introduit, comprenant les étapes de ;
(1) préparation d'un vecteur rétroviral comprenant une origine de réplication, une séquence A, un gène marqueur X, des séquences C et D destinées à introduire un gène étranger par recombinaison homologue, et une séquence B qui est homologue avec ladite séquence A, dans cet ordre, dans lequel lesdites séquences C et D sont directement ou indirectement adjacentes, et pour une utilisation dans l'introduction du gène étranger entre lesdites séquences adjacentes C et D ;
(2) préparation d'un fragment d'acide nucléique contenant une séquence C' qui est homologue avec ladite séquence C, un gène étranger, et une séquence D' qui est homologue avec ladite séquence D, dans cet ordre ;
(3) par exposition dudit vecteur rétroviral et dudit fragment d'acide nucléique à des conditions pour entraîner une recombinaison homologue, génération d'un vecteur rétroviral recombinant dans lequel le gène étranger a été introduit entre lesdites séquences adjacentes C et D, et formation d'un hôte qui contient le vecteur rétroviral recombinant dans lequel le gène étranger a été introduit entre lesdites séquences adjacentes C et D ;
(4) culture de l'hôte obtenu dans l'étape (3), et sélection, à partir de la culture, d'un hôte contenant le vecteur rétroviral recombinant dans lequel le gène étranger a été introduit entre lesdites séquences adjacentes C et D par identification de l'expression du gène marqueur X comme indicateur ; et
(5) extraction depuis l'hôte choisi dans l'étape (4), du vecteur rétroviral recombinant dans lequel le gène étranger a été introduit entre lesdites séquences adjacentes C et D,
dans lequel ladite séquence A est une longue répétition terminale (LRT) en 3' contenue dans un génome rétroviral et ladite séquence B est une (LRT) en 5' contenue dans un génome rétroviral, ou ladite séquence A est une (LRT) en 5' contenue dans un génome rétroviral et ladite séquence B est une (LRT) en 3' contenue dans un génome rétroviral.

2. Procédé selon la revendication 1, dans lequel ladite étape (3) est réalisée par mélange dudit vecteur rétroviral et dudit fragment d'acide nucléique avec une enzyme pour recombinaison homologue pour obtenir un produit de recombinaison homologue, et transformation d'un hôte avec le produit obtenu.

3. Procédé selon la revendication 1, dans lequel ladite étape (3) est réalisée par obtention d'un gène codant une enzyme pour recombinaison homologue et d'un hôte contenant ledit fragment d'acide nucléique et ledit vecteur rétroviral, et expression du gène codant une enzyme pour recombinaison homologue dans l'hôte obtenu.

4. Procédé selon les revendications 1 à 3, dans lequel le vecteur rétroviral est un vecteur lentiviral.

5. Procédé selon l'une quelconque des revendications 1 à 4, dans lequel le gène marqueur X est un gène de protéine de fluorescence ou un gène pharmacorésistant.

6. Procédé selon l'une quelconque des revendications 1 à 4, dans lequel le gène marqueur X est un gène codant la luciférase ou la β-galactosidase.

7. Procédé selon l'une quelconque des revendications 1 à 6, dans lequel l'origine de réplication et la séquence A sont indirectement adjacentes, et un gène marqueur Y différent du gène marqueur X est contenu entre ladite origine de réplication et la séquence A adjacentes.

8. Procédé selon l'une quelconque des revendications 1 à 6, dans lequel l'origine de réplication et la séquence B sont indirectement adjacentes, et un gène marqueur Y différent du gène marqueur X est contenu entre ladite origine de réplication et la séquence B adjacentes.

9. Procédé selon la revendication 7 ou 8, dans lequel le gène marqueur Y est un gène de protéine de fluorescence ou un gène pharmacorésistant.

10. Procédé selon la revendication 7 ou 8, dans lequel le gène marqueur Y est un gène codant la luciférase ou la β-galactosidase.

11. Procédé selon l'une quelconque des revendications 1 à 10, dans lequel l'origine de réplication est une origine de réplication d'un organisme choisi parmi *Escherichia coli,* un bactériophage, *Saccharomyces cerevisiae* et *Schizosaccharomyces pombe.*

12. Utilisation d'un vecteur rétroviral comprenant une origine de réplication, une séquence A, un gène marqueur X, des séquences C et D destinées à introduire un gène étranger par recombinaison homologue, et une séquence B qui est homologue avec ladite séquence A, dans cet ordre, dans laquelle lesdites séquences C et D sont directement ou indirectement adjacentes, et pour une utilisation dans l'introduction du gène étranger entre lesdites séquences adjacentes C et D dans un procédé selon les revendications 1 à 10,
dans laquelle ladite séquence A est une (LRT) en 3' contenue dans un génome rétroviral et ladite séquence B est une (LRT) en 5' contenue dans un génome rétroviral, ou ladite séquence A est une (LRT) en 5' contenue dans un génome rétroviral et ladite séquence B est une (LRT) en 3' contenue dans un génome rétroviral.


### SEQUENCE LISTING

<110> University of Toyama
<120> Vector for Introduction of Foreign Gene and Method of Producing
   Vector including Foreign Gene
<130> 125023H
<150> JP2011- 75273
   <151> 2011-03-30
<160> 4
<170> PatentIn version 3.5
<210> 1
   <211> 594
   <212> DNA
   <213> Lentivirus
<220>
   <223> Inventor: HORII, Masae; KISHI, Hiroyuki Inventor: KOBAYASHI, Eiji; OZAWA, Tatsuhiko; MURAGUCHI, Atsushi
<400> 1
   aatgaaagac cccacctgta ggtttggcaa gctagcttaa gtaacgccat tttgcaaggc     60
   atggaaaaat acataactga gaatagaaaa gttcagatca aggtcaggaa cagatggaac    120
   agctgaatat gggccaaagc ggatatctgt ggtaagcagt tcctgccccg gctcagggcc    180
   aagaacagat ggaacagctg aatatgggcc aaacaggata tctgtggtaa gcagttcctg    240
   ccccggctca gggccaagaa cagatggtcc ccagatgcgg tccagccctc agcagtttct    300
   agagaaccat cagatgtttc cagggtgccc caaggacctg aaatgaccct gtgccttatt    360
   tgaactaacc aatcagttcg cttctcgctt ctgttcgcgc gcttctgctc cccgagctca    420
   ataaaagagc ccacaacccc tcactcgggg cgccagtcct ccgattgact gagtcgcccg    480
   ggtacccgtg tatccaataa accctcttgc agttgcatcc gacttgtggt ctcgctgttc    540
   cttgggaggg tctcctctga gtgattgact acccgtcagc gggggtcttt catt           594
<210> 2
   <211> 594
   <212> DNA
   <213> Lentivirus
<400> 2
   aatgaaagac cccacctgta ggtttggcaa gctagcttaa gtaacgccat tttggaaggc      60
   atggaaaaat acataactga gaatagagaa gttcagatca aggtcaggaa cagatggaac     120
   agctgaatat gggccaaaca ggatatctgt ggtaagcagt tcctgccccg gctcagggcc     180
   aagaacagat ggaacagctg aatatgggcc aaacaggata tctgtggtaa gcagttcctg     240
   ccccggctca gggccaagaa cagatggtcc ccagatgcgg tccagccctc agcagtttct     300
   agagaaccat cagatgtttc cagggtgccc caaggacctg aaatgaccct gtgccttatt     360
   tgaactaacc aatcagttcg cttctcgctt ctgttcgcgc gcttctgctc cccgagctca     420
   ataaaagagc ccacaacccc tcactcgggg cgccagtcct ccgattgact gagtcgcccg     480
   ggtacccgtg tatccaataa accctcttgc agttgcatcc gacttgtggt ctcgctgttc     540
   cttgggaggg tctcctctga gtgattgact acccgtcagc gggggtcttt catt           594
<210> 3
   <211> 471
   <212> DNA
   <213> Homo sapiens
<220>
   <221> CDS
   <222> (1)..(471)
<400> 3
   atg aca tcc att cga gct gta ttt ata ttc ctg tgg ctg cag ctg gac       48
   Met Thr Ser Ile Arg Ala Val Phe Ile Phe Leu Trp Leu Gln Leu Asp
   1               5                   10                  15
   ttg gtg aat gga gag aat gtg gag cag cat cct tca acc ctg agt gtc       96
   Leu Val Asn Gly Glu Asn Val Glu Gln His Pro Ser Thr Leu Ser Val
               20                  25                  30
   cag gag gga gac agc gct gtt atc aag tgt act tat tca gac agt gcc      144
   Gln Glu Gly Asp Ser Ala Val Ile Lys Cys Thr Tyr Ser Asp Ser Ala
           35                  40                  45
   tca aac tac ttc cct tgg tat aag caa gaa ctt gga aaa aga cct cag      192
   Ser Asn Tyr Phe Pro Trp Tyr Lys Gln Glu Leu Gly Lys Arg Pro Gln
       50                  55                  60
   ctt att ata gac att cgt tca aat gtg ggc gaa aag aaa gac caa cga      240
   Leu Ile Ile Asp Ile Arg Ser Asn Val Gly Glu Lys Lys Asp Gln Arg
   65                  70                  75                  80
   att gct gtt aca ttg aac aag aca gcc aaa cat ttc tcc ctg cac atc      288
   Ile Ala Val Thr Leu Asn Lys Thr Ala Lys His Phe Ser Leu His Ile
                   85                  90                  95
   aca gag acc caa cct gaa gac tcg gct gtc tac ttc tgt gca gca agg      336
   Thr Glu Thr Gln Pro Glu Asp Ser Ala Val Tyr Phe Cys Ala Ala Arg
               100                 105                 110
   aaa gct gca ggc aac aag cta act ttt gga gga gga acc agg gtg cta      384
   Lys Ala Ala Gly Asn Lys Leu Thr Phe Gly Gly Gly Thr Arg Val Leu
           115                 120                 125
   gtt aaa cca aat atc cag aac cct gac cct gcc gtg tac cag ctg aga      432
   Val Lys Pro Asn Ile Gln Asn Pro Asp Pro Ala Val Tyr Gln Leu Arg
       130                 135                 140
   gac tct aaa tcc agt gac aag tct gtc tgc cta ttc acc                  471
   Asp Ser Lys Ser Ser Asp Lys Ser Val Cys Leu Phe Thr
   145                 150                 155
<210> 4
   <211> 157
   <212> PRT
   <213> Homo sapiens
<400> 4
   Met Thr Ser Ile Arg Ala Val Phe Ile Phe Leu Trp Leu Gln Leu Asp
   1               5                   10                  15
   Leu Val Asn Gly Glu Asn Val Glu Gln His Pro Ser Thr Leu Ser Val
               20                  25                  30
   Gln Glu Gly Asp Ser Ala Val Ile Lys Cys Thr Tyr Ser Asp Ser Ala
           35                  40                  45
   Ser Asn Tyr Phe Pro Trp Tyr Lys Gln Glu Leu Gly Lys Arg Pro Gln
       50                  55                  60
   Leu Ile Ile Asp Ile Arg Ser Asn Val Gly Glu Lys Lys Asp Gln Arg
   65                  70                  75                  80
   Ile Ala Val Thr Leu Asn Lys Thr Ala Lys His Phe Ser Leu His Ile
                   85                  90                  95
   Thr Glu Thr Gln Pro Glu Asp Ser Ala Val Tyr Phe Cys Ala Ala Arg
               100                 105                 110
   Lys Ala Ala Gly Asn Lys Leu Thr Phe Gly Gly Gly Thr Arg Val Leu
           115                 120                 125
   Val Lys Pro Asn Ile Gln Asn Pro Asp Pro Ala Val Tyr Gln Leu Arg
       130                 135                 140
   Asp Ser Lys Ser Ser Asp Lys Ser Val Cys Leu Phe Thr
   145                 150                 155